# EUROPEAN PATENT SPECIFICATION

(11) **EP 2 054 852 B1**
(45) Date of publication and mention of the grant of the patent: **23.06.2010**
(21) Application number: 07837227.3
(22) Date of filing: 21.08.2007
(51) Int. Cl.: G06T 5/00

(54) **COMPUTER AIDED ANALYSIS USING VIDEO FROM ENDOSCOPES**
COMPUTERGESTÜTZTE ANALYSE MIT HILFE VON VIDEODATEN AUS ENDOSKOPEN
ANALYSE ASSISTÉE PAR ORDINATEUR UTILISANT UNE VIDÉO ISSUE D'ENDOSCOPES

(30) Priority: 21.08.2006 US 839275 P
(43) Date of publication of application: 06.05.2009
(73) Proprietor: STI Medical Systems, LLC, Honolulu, HI 96813 (US)
(72) Inventor: GU, Jia, Honolulu, HI 96826 (US); WOLTERS, Rolf, Holger, Kailua, HI 96734 (US)
(74) Representative: Robinson, Ian Michael
(86) International application number: PCT/US2007/018600
(87) International publication number: WO 2008/024419

(56) References cited:
- WO-A-2005/062253
- US-A1- 2003 208 107
- J. GU, W. LI: "Automatic Image Quality Assessment for Uterine Cervical Imagery" PROCEEDINGS OF SPIE, [Online] vol. 6146, 17 March 2006 (2006-03-17), XP002463547 Retrieved from the Internet: URL:spiedigitallibrary.aip.org/getpdf/serv let/GetPDFServlet?filetype=pdf&id=PSISDG00 614600000161461B000001&idtype=cvips&prog=n ormal> [retrieved on 2007-12-21] cited in the application
- T. THORMÄLEN, H. BROSZIO, P. N. MEIER: "Three-Dimensional Endoscopy" FALK SYMPOSIUM NO. 124, MEDICAL IMAGING IN GASTROENTEROLOGY AND HEPATOLOGY, [Online] 2002, XP002463548 Kluwer Academic Publishers, Hannover ISBN: 0-7923-8774-0 Retrieved from the Internet: URL:ftp.tnt.uni-hannover.de/pub/papers/200 2/FALK124_TTHBPM.pdf> [retrieved on 2007-12-21]
- KUDO ET AL: "Diagnosis of colorectal tumorous lesions by magnifying endoscopy" GASTROINTESTINAL ENDOSCOPY, ELSEVIER, NL, vol. 44, no. 1, July 1996 (1996-07), pages 8-14, XP005127697 ISSN: 0016-5107 cited in the application

## Description

### Technical Field.

This invention generally relates to medical imaging and more specifically to image processing and computer aided diagnosis for diseases, such as colorectal cancer, using an automated image processing system providing a rapid, inexpensive analysis of video from a standard endoscope, optionally including a 3 dimensional ("3D") reconstructed view of the organ of interest, such as a patient's colon. This invention is to be used in real time employing video data from a conventional endoscope that is already being used during an examination, such as a colonoscopy, to provide an instantaneous second opinion without substantially prolonging the examination.

### Background Art.

Although this invention is being disclosed in connection with colorectal cancer, it is applicable to many other areas of medicine. Colorectal cancer is the second leading cause of cancer-related deaths in the United States. More than 130,000 people are diagnosed with colon cancer each year and about 55,000 people die from the disease annually. Colon cancer can be prevented and cured through early detection, so early diagnosis is of critical importance for patient survival (American Cancer Society, Cancer Facts and Figures, 2004).

Screening for polyps using an endoscope is the current and most suitable prevention method for early detection and removal of colorectal polyps. If such polyps remain in the colon, they can possibly grow into malignant lesions (Hofstad, B., Vatn, M.H., Andersen, S.N., Huitfeldt, H.S. et al., Growth of colorectal polyps: redetection and evaluation of unresected polyps for a period of three years, Gut 39(3): 449-456. 1996). In the case of flat lesions, in which no protruding polyps are present, the colonic mucosal surface is granular and demarcated into small areas called nonspecific grooves. Changes in the cellular pattern (pit pattern) of the colon lining might be the very earliest sign of adenoma or tumors (Muehldorfer, S.M., Muenzenmayer, C., Mayinger, B., Faller, G. et al., Optical tissue recognition based on color texture by magnifying endoscopy in patients with Barrett's esophagus, Gastrointestinal Endoscopy 57: AB179, 2003). Pit patterns can be used for a qualitative detection of lesions to measure these textural alterations of the colonic mucosal surface. Though the specificity using pit patterns is low, its relatively high sensitivity can highlight suspicious regions, permitting further examination by other sensors. Texture-based pit-pattern analysis can identify tissue types and disease severity. Image-based polyp reconstruction can provide 3 dimensional shape and size of a protruding polyp, using video from an endoscope and computer vision algorithms to synthesize multiple-views that can be converted into 3 dimensional images. Optionally, several image processing algorithms and enhancement techniques can be used to improve image quality.

Various non-invasive scanning techniques have been proposed to avoid the need for a colonoscopy, but if these scanning techniques disclose the possible existence of a polyp or lesion, a colonoscopy must be performed later anyway. Further, the colonoscopist must locate the actual polyp or lesion (which was shown in the scan) in the patient's colon at a remote time after the scan, which can be very difficult. Also, scans do not provide information about the color or texture of the interior surface of the colon, which would provide diagnostic information about vessel structure and pit patterns, especially for flat lesions. It is highly desirable to avoid false positives for flat lesions because they do not project outward from the colon wall, so that they must be removed by cutting into the colon wall, thus incurring greater risks of bleeding, infection and other adverse side effects. Also, scans may not be able to differentiate between polyps or lesions and residual stool or other material in the colon.

Colonoscopies must be done efficiently in order to be economical, so the colonoscopist must rapidly scan the comparatively large area of the interior surface of the large intestine. Accordingly, there is a risk that a lesion or polyp may be overlooked.

If a lesion or polyp is found during a colonoscopy, it is unnecessary to relocate it in a later procedure because the endosclope is already at the lesion or polyp and most endoscopes are equipped with a means by which to introduce cutting instruments. Thus, a polyp or lesion can be cut out during the same colonoscopy in which it was detected, either at the time it was first detected, or at a later time during the same colonoscopy.

The skill of a colonoscopist in detecting and analyzing polyps and lesions depends on the individual colonoscopist's training and experience. Thus, to standardize detection and analysis in colonoscopies, it is desirable to provide independent expert analysis in real time during a colonoscopy to alert the colonoscopist to a potential polyp or lesion, or to confirm or question a diagnosis of any polyp or lesion that is found. It is also desirable to provide such expert knowledge in an inexpensive and readily available manner, without requiring the purchase of additional expensive hardware.

US2003/208107A1 discloses an encapsulated medical imaging device which is swallowed by a patient and passes through their gastrointestinal tract by natural action. The device includes a plurality of microlenses which each have a different focal length. A focused image from a microlens whose focal distance matches the distance of the device from the intestinal wall is selected and displayed, whereas unfocused data is discarded.

### DISCLOSURE OF INVENTION

According to the present invention there is provided an apparatus as set forth in the appended claims. Other features of the invention will be apparent from the dependent claims, and the description which follows.

### Brief Description of Drawings.

FIG. 1 shows the algorithm flowchart of a computer aided diagnosis system.
FIG. 2 shows the algorithm flowchart of the video quality analysis module of Fig. 1.
FIG. 3 shows the algorithm flowchart of the video quality enhancement module of Fig. 1.
FIG. 4 shows the algorithm flowchart of the glint detection and elimination module of Fig. 2.
FIGS. 5A and 5B show the results of glint detection and elimination, with FIG. 5A showing the original image frame, and FIG. 5B showing glint removed from the image.
FIG. 6 shows the algorithm flowchart of blur detection.
FIGS. 7A and 7B show the results of blur detection, with FIG. 7A showing the original image frame, and FIG. 7B showing the results of blur detection (the black blocks overlapped on the image indicate the location of the blurry area).
FIGS. 8A and 8B show the results of contrast enhancement, with FIG. 8A showing the original image frame, and FIG. 8B showing the contrast enhanced image.
FIGS. 9A, 9B, 9C, 9D and 9E show the results of super-resolution reconstruction, with FIGS. 9A, 9B. 9C and 9D showing four frames of low resolution images, and FIG. 9E showing a reconstructed higher resolution image
FIG. 10 shows the algorithm flowchart of video stabilization.
FIGS. 11A and 11B show the results of video stabilization, with FIGS. 11A and 11B showing motion fields of two colonoscopic videos (the curves with circles representing the motion field of original video with shaky movement, and the curves with squares representing the motion field of stabilized video).
FIG. 12 shows the algorithm flowchart of 3D colon modeling and reconstruction.
FIGS. 13A, 13B, 13C and 13D show the results of distortion correction, with FIG. 13A showing the original image of a grid target, FIG. 13B showing the corrected grid target, FIG. 13C showing the original image of a colon, and FIG. 13D showing the corrected colon image.
FIGS. 14A, 14B, 14C and 14D show the results of 3D polyp reconstruction, with FIGS. 14A and 14B showing two colonoscopic video frames, FIG. 14C showing a reconstructed 3D polyp model, and FIG. 14D showing a 3D polyp visualization with texture mapping.
FIG. 15 shows the Kudo pit pattern classification patterns.
FIG. 16 shows the algorithm flowchart of color analysis.
FIG. 17 shows the algorithm flowchart of texture analysis.
FIGS. 18A, 18B and 18C show the results of pit pattern extraction and identification, with FIG. 18A showing the original image, FIG. 18B showing image enhancement results, and FIG. 18C showing statistical information from the automated algorithm, in which numbers indicate the pit size, and the arrowed broken circles represents round pit shape, and "+"represents elongated pit shape. In this example, the algorithm identifies the tissue as Kudo IV pit, which matches the ground-truth.

### Best Modes for Carrying Out Invention.

### 1. System framework of automatic image quality assessment

The present invention is a complex multi-sensor, multi-data and multi-algorithm image processing system. The design provides a modular and open architecture built on phenomenology (feature) based processing. The feature set includes the same features used by the colonoscopists to assess the disease severity (polyp size, pit pattern, etc.). The image-based polyp reconstruction algorithm features several steps: distortion correction, image based modeling, 3D data stitching and reconstruction. The texture-based pit-pattern analysis employs morphological operators to extract the texture pattern, and then utilizes a statistical model and machine learning algorithms to classify the disease severity according to the color and texture information of pits. By analyzing the 3D poly shape and pit-pattern the colonoscopist is provided with diagnostic information for macroscopic inspection. The open architecture also allows for a seamless integration of additional features (Maroulis, D.E., Iakovidis, D.K., Karkanis, S.A., and Karras, D.A., CoLD: A versatile detection system for colorectal lesions in endoscopy video-frames, Compute Methods Programs Biomed. 70(2): 151-166. 2003; Buchsbaum, P.E. and Morris, M.J., Method for making monolithic patterning dichroic filter detector arrays for spectroscopic imaging, Ocean Optics, Inc., US Patent# 6,638,668 Barrie, J.D., Aitchison, K.A., Rossano, G.S., and Abraham, M.H., Patterning of multilayer dielectric optical coatings for multispectral CCDs, Thin Solid Films 270: 6-9, 1995) from other microscopic modalities (such as OCT: Optical Coherence Tomography, FTIR: Fourier Transform Infrared and Confocal Microscopy), to provide more accurate diagnostic information. Our system also allows for visualization and virtual navigation for Colonoscopist, and this is done by virtual reality techniques and a magnetic sensor, which provides us absolute spatial location and orientation.

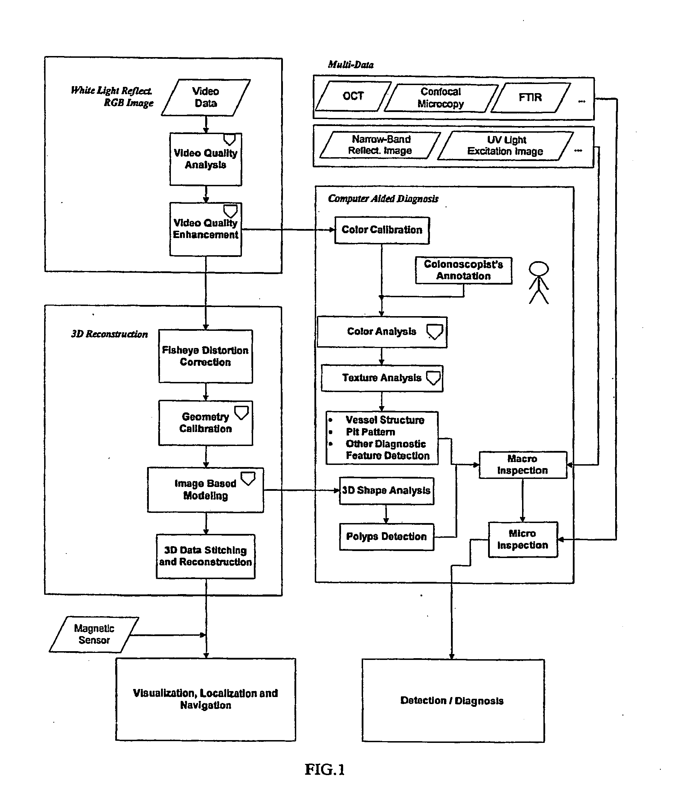

The system described in this invention starts from RGB (Red-Green-Blue color space) videos acquired from a digital endoscope. A series of algorithms is employed to perform the image preprocessing (Pascale, D., A Review of RGB Color Spaces, 5700 Hector Desloges, Montreal (Quebec), Canada, the Babel Color Company. 2003; Wolf, S., Color Correction Matrix for Digital Still and Video Imaging Systems, NTIA Technical Memorandum TM-04-406. 2003), and this is done by two modules, the first being the video quality analysis module, which aims to discard poor quality image frames and delete them from the video; the second being the video quality enhancement module, which aims to improve the image quality and reduce image artifacts. The whole framework is shown in FIG. 1.

### 2. Video quality analysis and enhancement

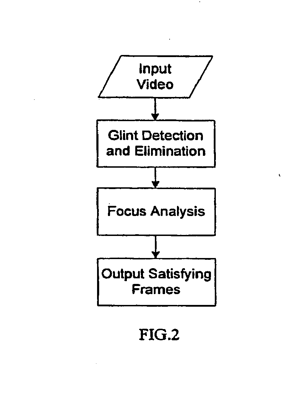

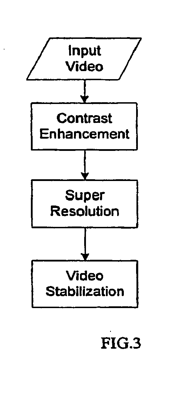

The video quality analysis and enhancement comprises a glint removal algorithm, a blur detection algorithm, a contrast enhancement algorithm, a super-resolution reconstruction algorithm and a video stabilization algorithm. The framework is shown in FIG. 2 and FIG. 3.

### 2.1 Glint removal algorithm

We incorporate the same glint removal algorithm that we designed for cervical cancer CAD.

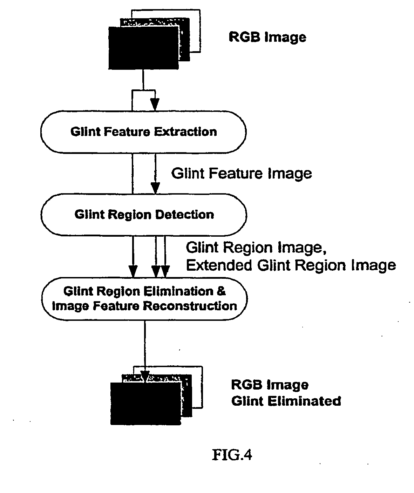

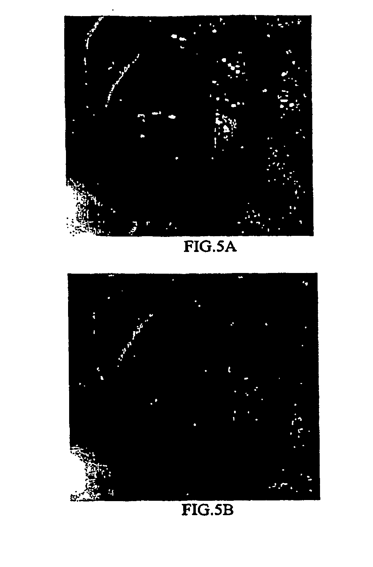

(Lange H.; Automatic glare removal in reflectance imagery of the uterine cervix; SPIE Medical Imaging 2005; SPIE Proc. 5747, 2005). The method is to extract a glint feature signal from the RGB image that provides a good glint to background ratio, finds the glint regions in the image, and then eliminates the glint regions by restoring the estimated image features for those regions. We have chosen the G (Green) image component as the glint feature signal, because it provides a high glint to background ratio and simplicity of calculation. Glint regions are either detected as saturated regions or small high contrasted regions. Saturated regions are detected using an adaptive thresholding method. Small high contrasted bright regions are detected using morphological top hat filters with different sizes and thresholds. The full extent of the glint regions are approximated using a morphological constraint watershed segmentation algorithm plus a constant dilation. The image features (R,G,B) are first interpolated from the surrounding regions based on Laplace's equation. Then the intensity image feature is restored by adding to the interpolated region intensity function a scaled intensity function that is based on the error function between the interpolated region intensity and the raw intensity data from the region and a signal based on the detected binary glint region. The glint detection and elimination algorithm consists of three consecutive processing steps: (1) Glint feature extraction, (2) Glint region detection, and (3) Glint region elimination and image feature reconstruction. FIG. 4 shows the algorithm flowchart of the glint detection and elimination algorithm, and the results of the glint detection and elimination algorithm can be viewed in FIGS. 5A and 5B.

### 2.2 Blur detection algorithm

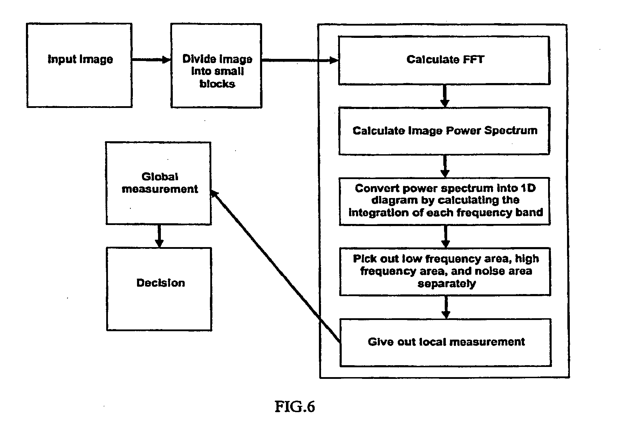

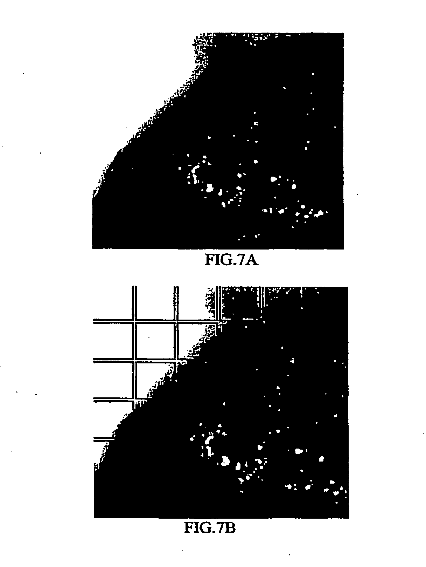

The blur detection algorithm utilizes a normalized image power spectrum method (Gu J. and Li W., Automatic Image Quality Assessment for Cervical Imagery, SPIE Medical Imaging 2006; SPIE Proc. 6146, 2006), which can be described as the following steps:
1. Divide the image into non-overlapping blocks.
2. For each block, compute local representatives based on frequency information.
3. Compute global statistics from local representatives obtained from Step 2.
4. Determine whether the image is blurred or not from the global statistics. The local representative is calculated by image power spectrum, and then it is normalized by the zero components. Afterward, this 2D image power spectrum is transformed into a 1D diagram. In order to analyze the energy property in each frequency band, polar coordinate integration is used according to each radial value. The power spectrum is separated into three parts and the low frequency area is considered to represent structure information invariant to blur, and the high frequency area is considered to represent detailed information, which is more sensitive to blur. By analyzing the ratio between these two integrations, the degree of blur can be calculated (the noise spectrum has been discarded previously by a threshold). After the blur degree of each small block has been determined, the global measurement a decision can be made as a whole. This can be done by using the percentage of the numbers of blurred blocks occupied in the entire image. Furthermore, different weights are given between those blocks in the center and those blocks in the periphery, since the quality of the image center is of more concern. Thus, if the coverage of blurred blocks is less than a certain threshold, the image is deemed to be satisfyingly clear, otherwise an error message will pop up and feedback to the operator as a blurred image. FIG. 6 shows the algorithm flowchart and the result of blur detection can be seen in FIGS. 7A and 7B.

### 2.3 Contrast enhancement

The method preferably used for contrast enhancement is adaptive histogram equalization. Adaptive histogram equalization enhances the contrast of images by transforming the values in the intensity image (Zuiderveld, K., Contrast limited adaptive histogram equalization, Princeton, NJ: Academic Press, Graphics gems IV, ed. Heckbert, P., 1994).

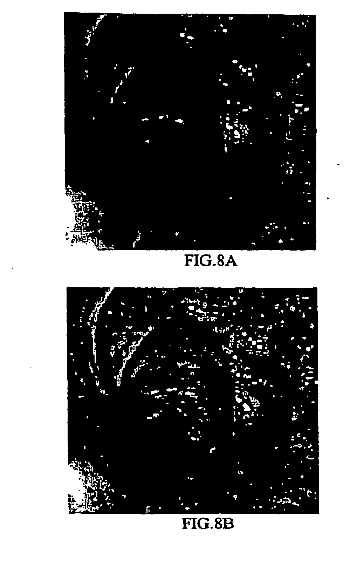

Unlike global histogram equalization, adaptive histogram equalization operates on small data regions (windows), rather than the entire image. Each window's contrast is enhanced, so that the histogram of the output region approximately matches the specified histogram. The neighboring windows are then combined using bilinear interpolation in order to eliminate artificially induced boundaries. The contrast, especially in homogeneous areas, can be limited in order to avoid amplifying the noise which might be present in the image. The results of contrast enhancement can be viewed in FIGS. 8A and 8B.

### 2.4 Super-resolution reconstruction

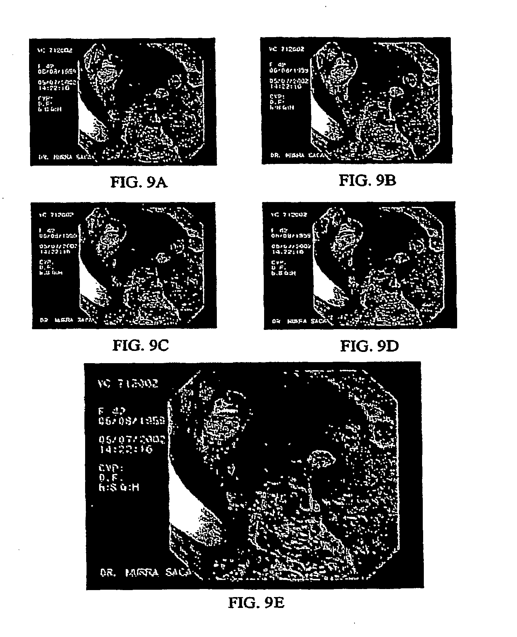

Super resolution is a technique to use multiple frames of the same object to achieve a higher resolution image (Kim, S.P., Bose, N.K, and Valensuela, H.M., Recursive reconstruction of high resolution image from noisy undersampled multiframes, IEEE Transaction on Acoustics, Speech, and Signal Processing 38(6): 1031-1027, 1990; Irani, M. and Peleg, S., Improving resolution by image registration, CVGIP:GM 53: 231-239, 1991). Super resolution works when the frames are shifted by fractions of a pixel from each other. The super-resolution algorithm is able to produce a larger image that contains the information in the smaller original frames, first an image sub-pixel registration is employed to establish the correspondence between several low resolution images, and then a sub-pixel interpolation algorithm is used to reconstruct the higher resolution image. FIGS. 9A, 9B, 9C, 9D and 9E show a super-resolution reconstruction result.

### 2.5 Video stabilization

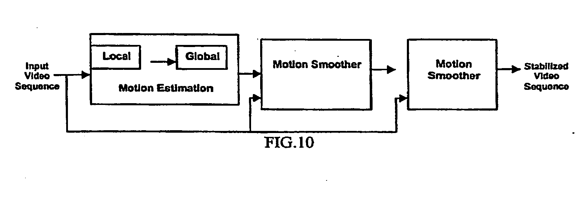

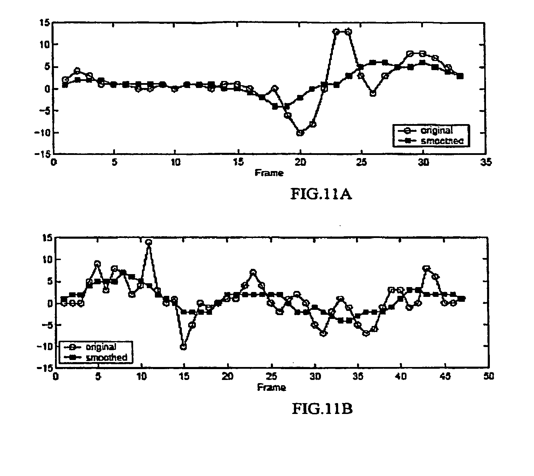

Video stabilization is the process of generating a compensated video sequence by removing image motion from the camera's undesirable shake or jiggle. The preferred video stabilization algorithm consists of a motion estimation (ME) block, a motion smooth (MS) block, and a motion correction (MC) block, as shown in FIG. 10. The ME block estimates the motion between frames and can be divided into a local motion estimator and a global motion decision unit. The local motion estimator will return the estimated dense optical flow information between successive frames using typical block-based or gradient-based methods. The global motion decision unit will then determine an appropriate global transformation that best characterizes the motion described by the given optical flow information. The global motion parameters will be sent to the MS, where the motion parameters are often filtered to remove the unwanted camera motion but retaining intentional camera motion. Finally MC warps the current frame using the filtered global transformation information and generates the stabilized video sequence. FIGS. 11A and 11B show the result of video stabilization.

### 3. Three dimensional colon modeling and reconstruction

A 3D colon model is a preferred component of a computer-aided diagnosis (CAD) system in colonoscopy, to assist surgeons in visualization, and surgical planning and training. The ability to construct a 3D colon model from endoscopic videos (or images) is thus preferred in a CAD system for colonoscopy. The mathematical formulations and algorithms have been developed for modeling static, localized 3D anatomic structures within a colon that can be rendered from multiple novel view points for close scrutiny and precise dimensioning (Mori, K., Deguchi, D., Sugiyama, J., Suenaga, Y. et al., Tracking of a bronchoscope using epipolar geometry analysis and intensity-based image registration of real and virtual endoscopic images, Med. Image Anal. 6(3): 321-336. 2002; Lyon, R. and Hubel, P., Eyeing the camera: into the next century, 349-355. IS&T/TSID 10th Color Imaging Conference, Scottsdale, AZ, 2002; Zhang, X. and Payandeh, S., Toward Application of Image Tracking in Laparoscopic Surgery, in International Conference on Pattern Recognition, Proc. of International Conference on Pattern Recognition 364-367. ICPR2000, 2000. This ability is useful for the scenario when a surgeon notices some abnormal tissue growth and wants a closer inspection and precise dimensioning.

The modeling system of the present invention uses only video images and follows a well-established computer-vision paradigm for image-based modeling. Prominent features are extracted from images and their correspondences are established across multiple images by continuous tracking and discrete matching. These feature correspondences are used to infer the camera's movement. The camera motion parameters allow rectifying of images into a standard stereo configuration and inferring of pixel movements (disparity) in these images. The inferred disparity is then used to recover 3D surface depth. The inferred 3D depth, together with texture information recorded in images, allow constructing of a 3D model with both structure and appearance information that can be rendered from multiple novel view points. More precisely, the modeling system comprises the following components:
1. Calibration: This is an offline process to estimate intrinsic camera parameters and to correct image distortion (e.g., lens distortion), if any,
2. Feature selection: This step identifies unique and invariant colon features for ensuing video analysis,
3. Feature matching: This step matches image features across multiple images or video frames to establish correspondences of these features,
4. Camera motion Inference: This step uses matched image features to infer camera movement between adjacent images or video frames,
5. Image rectification: This step is to rearrange image pixels in such a way that corresponding epipolar lines from two images are aligned and stacked as image scan lines. This step allows standard stereo matching techniques to be applicable regardless of camera movement,
6. Stereo matching: This step is to compute disparity (image movement) between two rectified images to allow 3D depth inference,
7. Model construction: This step is to infer 3D depth from pixel disparity and construct a 3D model that captures both structure and appearance information, and
8. Model rendering: This final step is for rendering the computer model from any novel view points.

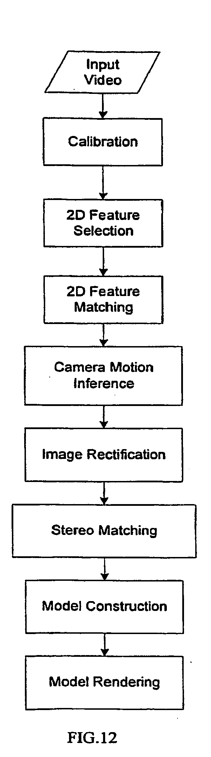

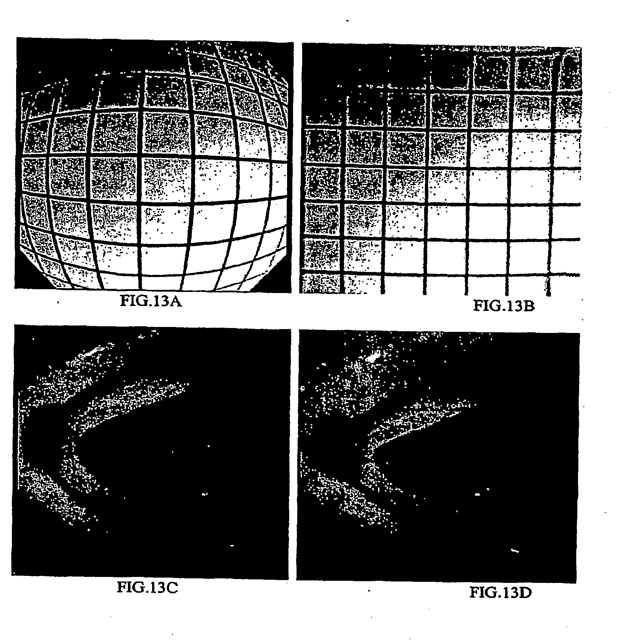

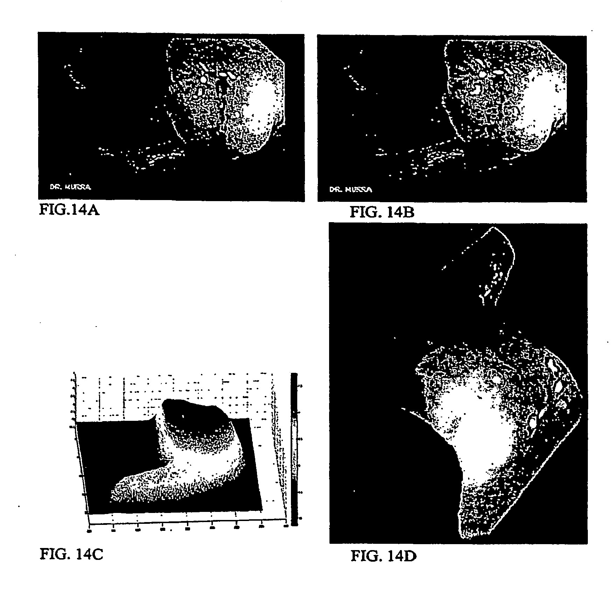

FIG. 12 shows the algorithm flowchart of 3D colon modeling and reconstruction. FIGS. 13A, 13B, 13C and 13D show the distortion correction result and FIGS 14A, 14B, 14C and 14D show a result of 3D polyp reconstruction.

Creating a three-dimensional polyp model from endoscopic videos allows accomplishment of three goals. First, the model will allow the clinician to mark areas (on the model) during the entry phase of the colonoscopic exam, and to treat these areas during withdrawal. Second, for high-risk patients that require surveillance, it provides a framework for registering the patient's clinical state across exams, thereby enabling change detection. Third, after the 3D reconstruction, the system of this invention can quantitatively calculate the physical size of the polyp, and send to the colonoscopist a statistical criterion for making diagnostic decisions.

### 4. Pit pattern analysis

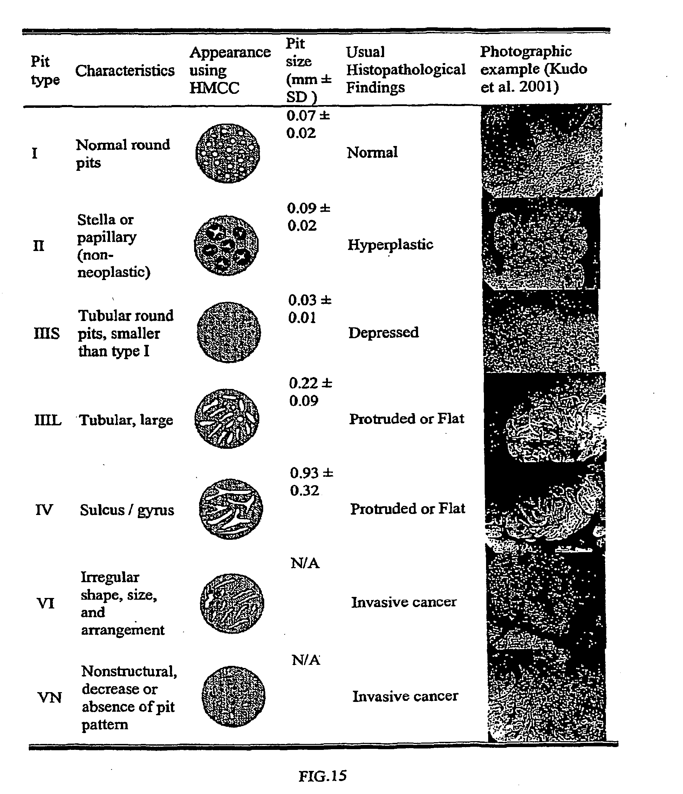

Treatment decisions for flat and depressed lesions are based on a detailed examination of the macroscopic morphological appearance, including the luminal surface structure of the crypts of Lieberkühn, otherwise known as "pit patterns". Pit patterns analysis can offer a degree of positive predictive value for both the underlying histology and depth of vertical submucosal invasion. The preferred system utilizes the Kudo tissue classification method which describes seven types of pit patterns (Kudo, S., Hirota, S., Nakajima, T., Hosobe, S., Kusaka, H., Kobayashi, T. et al., Colorectal tumours and pit pattern, Journal of Clinical Pathology. 47(10): 880-885. 1994. Kudo, S., Rubio, C.A., Teixeira, C.R., Kashida, H., and Kogure, E., Pit pattern in colorectal neoplasia: endoscopic magnifying view, Endoscopy 33(4): 367-373. 2001. Kudo, S., Tamura, S., Nakajima, T., Yamano, H., Kusaka, H., and Watanabe, H., Diagnosis of colorectal tumorous lesions by magnifying endoscopy, Gastrointestinal Endoscopy. 44(1): 8-14. 1996), according to histological, macroscopic morphology and size. These pit patterns have been correlated with histopathology to relate surface patterns to the underlying tissue structure. Lesions can be categorized into basic clinical groups: Kudo crypt group I/II constitutes non-neoplastic, non-invasive patterns. Group IIII/IIIS/IV/VI represents neoplastic but non-invasive lesions. Group VN represents neoplasia with accompanying invasive characteristics. Detailed characteristics are stated as follows, and appearances and photographic examples can be seen in FIG 15.
- The type I pit pattern of normal mucosa consists of rounded pits with a
- regular size and arrangement.
- The type II pit pattern of benign, hyperplastic polyps are larger than type I, and consist of relatively large star-like or onion-like pits.
- The type IIIL pit pattern is composed of tubular or rounded pits larger than type I, and is associated with polypoid adenomas.
- The type IIIS pit pattern is composed of tubular or rounded pits that are smaller than type I, and is associated with depressed lesions that are frequently high-grade dysplasia.
- The type IV pit pattern is a branched or gyrus-like pattern than is associated with adenomatous lesions.
- The type V pit pattern is divided into VI and VN. Type VI (irregular) has pits that are irregular in shape, size, and arrangement. Type VN (non-structural) shows an absence of pit pattern.

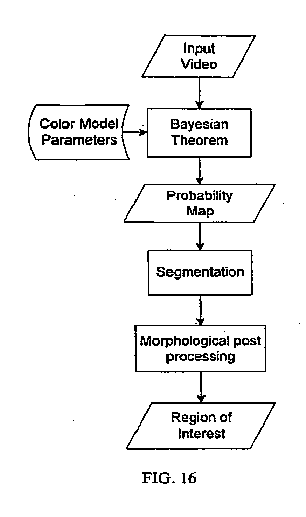

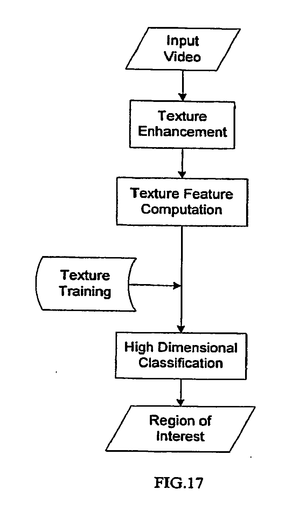

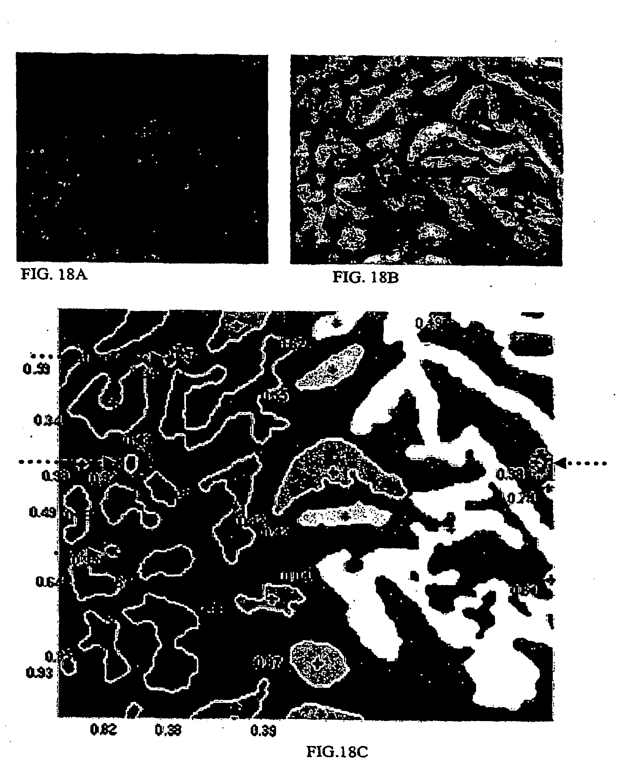

The pit pattern analysis module starts from high magnification endoscopic images, and morphological operators are first preformed to extract the texture pattern (Chen, C.H., Pau, L.F., and Wang, P.S.P., Segmentation Tools In Mathematical Morphology, Handbook of Pattern Recognition and Computer Vision, in Handbook of Pattern Recognition and Computer Vision, pp. 443-456. World Scientific Publishing Co., 1989). A statistical model and machine learning algorithms are then utilized (Sonka, M., Image Processing Analysis and Machine Vision, in 1998) to classify the disease severity according to the color and texture information (Lin, H.-C., Wang, L.-L., and Yang, S.-N., Extracting periodicity of a regular texture based on autocorrelation functions, Patter Recognition Letters 18(5): 433-443, ELSVIER Science Direct. 1997; Argenti, F., Alparone, L., and Benelli, G., Fast algorithms for texture analysis using co-occurrence matrices, in Radar and Signal Processing, IEE Proceedings F, 137: 443-448. [6], 1990) of pits. A large number of reference images with labeled annotations are used for training purpose, and thus the test images can be clustered against these reference imabased on the likelihood to different classes (Magoulas, G.D., Plagianakos, V.P., and Vrahatis, M.N., Neural network-based colonoscopic diagnosis using on-line learning and differential evolution, Applied Soft Computing 4(4): 369-379, 2004; Liu, Y., Collins, R.T., and Tsin, Y., A computational model for periodic pattern perception based on frieze and wallpaper groups, Liu, Y., Collins, R.T., and Tsin, Y., Pattern Analysis and Machine Intelligence, IEEE Transactions on 26(3): 354-371, 2004; Liu, Y., Lazar, N., Rothfus, W.E., Dellaert, F., Moore, S., Scheider, J., and Kanade, T., Semantic Based Biomedical Image Indexing and Retrival, Trends in Advances in Content-Based Image and Video Retrival, eds. Shapiro, D., Kriegel, and Veltkamp, 2004; Zhang, J., Collins, R., and Liu, Y., Representation and matching of articulated shapes, in Computer Vision and Pattern Recognition, 2: II-342-II-349.IEEE Conference on Computer Vision and Pattern Recognition CVPR 2004, 2004). The training feature includes: pit size, pit shape and pit density. FIG. 16 shows the algorithm flowchart of color analysis. FIG. 17 shows the algorithm flowchart of texture analysis. And FIG. 18 shows the result of pit pattern extraction and identification.

Although a few preferred embodiments have been shown and described, it will be appreciated by those skilled in the art that various changes and modifications might be made without departing from the scope of the invention, as defined in the appended claims.

### Industrial Applicability

This invention can be used whenever video data from an endoscope is available during an examination of an organ, and especially when it is necessary to diagnose potentially cancerous regions, and motion pictures of the regions are available.

## Claims

1. An automated image processing system comprising:
a computer coupled to an endoscope to receive video data during an examination by colonoscopy of a large intestine;
a video quality analysis module arranged to analyze said video data to determine image frames in which the coverage of blurred blocks is less than a threshold to provide satisfying image frames from said video data, and wherein said video quality analysis module is arranged to perform a glint removal algorithm which performs glint feature extraction, glint region detection and glint region elimination and image feature reconstruction;
a video quality enhancement module arranged to enhance contrast in said satisfying image frames;
apply super resolution to said satisfying image frames by using multiple frames of the same object to achieve a higher resolution image; and
provide video stabilization to said satisfying image frames; and
an analysis module arranged to detect and classify any lesions or polyps in said satisfying image frames to output diagnostic information regarding said lesions or polyps;
wherein said video quality analysis module, said video quality enhancement module, and said analysis module all operate to output the diagnostic information in real time during the examination.

2. The system according to claim 1, wherein said analysis module is arranged to perform color calibration, color analysis, texture analysis and feature detection.

3. The system according to claim 1, wherein said analysis module is arranged to perform analysis of blood vessels in the satisfying image frames.

4. The system according to claim 1, wherein said analysis module includes a pit pattern analysis module arranged to perform analysis of pit patterns in the satisfying image frames.

5. The system according to claim 1, further comprising a three-dimensional modelling and reconstruction module arranged to:
reconstruct a still image of said large intestine in three dimensions from said satisfying image frames to form a reconstructed model, wherein said reconstructing is performed in real time during said colonoscopy
perform fisheye distortion correction, geometry calibration, image based modelling and three dimensional data stitching; and
wherein said analysis module is further arranged to recover three-dimensional shape and size information of any lesions or polyps in said large intestine from said reconstructed model.

## Patentansprüche

1. Automatisiertes Bildverarbeitungssystem, umfassend:
einen Computer, der mit einem Endoskop gekoppelt ist, um während einer Untersuchung durch Koloskopie eines Dickdarms Videodaten zu empfangen;
ein Videoqualitätsanalysemodul, das dafür ausgelegt ist, die Videodaten zu analysieren, um Einzelbilder zu bestimmen, in denen die Abdeckung von unscharfen Blöcken kleiner als eine Schwelle ist, um zufriedenstellende Einzelbilder aus den Videodaten bereitzustellen, und wobei das Videoqualitätsanalysemodul dafür ausgelegt ist, einen Schimmerentfernungsalgorithmus auszuführen, der Schimmermerkmalextraktion, Schimmerregionendetektion und Schimmerregionenbeseitigung und Bildmerkmalrekonstruktion ausführt;
ein Videoqualitätsverbesserungsmodul, das für Folgendes ausgelegt ist:
Verbessern des Kontrasts in den zufriedenstellenden Einzelbildern;
Anwenden von Superauflösung auf die zufriedenstellenden Einzelbilder durch Verwendung mehrerer Bilder desselben Objekts, um ein höher auflösendes Bild zu erzielen; und
Bereitstellen von Videostabilisierung an den zufriedenstellenden Einzelbildern; und
ein Analysemodul, das dafür ausgelegt ist, jegliche Läsionen oder Polypen in den zufriedenstellenden Einzelbildern zu detektieren und zu klassifizieren, um diagnostische Informationen in Bezug auf die Läsionen oder Polypen auszugeben;
wobei das Videoqualitätsanalysemodul, das Videoqualitätsverbesserungsmodul und das Analysemodul alle wirken, um die diagnostischen Informationen während der Untersuchung in Echtzeit auszugeben.

2. System nach Anspruch 1, wobei das Analysemodul dafür ausgelegt ist, Farbkalibration, Farbanalyse, Texturanalyse und Merkmaldetektion auszuführen.

3. System nach Anspruch 1, wobei das Analysemodul dafür ausgelegt ist, eine Analyse von Blutgefäßen in den zufriedenstellenden Einzelbildern auszuführen.

4. System nach Anspruch 1, wobei das Analysemodul ein Pit-Pattern-Analysemodul umfasst, das dafür ausgelegt ist, eine Analyse von Pit-Pattern in den zufriedenstellenden Einzelbildern auszuführen.

5. System nach Anspruch 1, ferner umfassend ein Modul der dreidimensionalen Modellierung und Rekonstruktion, das für Folgendes ausgelegt ist:
Rekonstruieren eines Standbildes des Dickdarms in drei Dimensionen aus den zufriedenstellenden Einzelbildern, um ein rekonstruiertes Modell zu bilden, wobei das Rekonstruieren in Echtzeit während der Koloskopie ausgeführt wird,
Ausführen einer Fischaugenverzerrungskorrektur, Geometriekalibration, auf Bildern basierenden Modellierung und eines dreidimensionalen Daten-Stitching, und
wobei das Analysemodul ferner dafür ausgelegt ist, dreidimensionale Form- und Größeninformationen jeglicher Läsionen oder Polypen in dem Dickdarm aus dem rekonstruierten Modell wiederzugewinnen.

## Revendications

1. Système de traitement d'images automatisé comprenant :
un ordinateur couplé à un endoscope pour recevoir des données vidéo au cours d'un examen par colonoscopie d'un gros intestin ;
un module d'analyse de la qualité de la vidéo, arrangé pour analyser lesdites données vidéo pour déterminer les trames d'images dans lesquelles la couverture des blocs flous est inférieure à un seuil afin de délivrer des trames d'images satisfaisantes à partir desdites données vidéo, et où ledit module d'analyse de la qualité de la vidéo est arrangé pour exécuter un algorithme de suppression de la scintillation qui procède à l'extraction de la caractéristique de scintillation, à la détection de la région de scintillation et à l'élimination de la région de scintillation et à la reconstruction de la caractéristique de l'image ;
un module d'amélioration de la qualité de la vidéo arrangé pour :
améliorer le contraste dans lesdites trames d'images satisfaisantes ;
appliquer une super résolution auxdites trames d'images satisfaisantes en utilisant plusieurs trames du même objet pour obtenir une image de résolution plus élevée ; et
assurer la stabilisation vidéo desdites trames d'images satisfaisantes ; et
un module d'analyse arrangé pour détecter et classer toutes lésions ou tous polypes dans lesdites trames d'images satisfaisantes pour délivrer des informations de diagnostic concernant lesdites lésions ou lesdits polypes ;
dans lequel, ledit module d'analyse de la qualité de la vidéo, ledit module d'amélioration de la qualité de la vidéo et ledit module d'analyse fonctionnent tous pour délivrer les informations de diagnostic en temps réel pendant l'examen.

2. Système selon la revendication 1, dans lequel ledit module d'analyse est arrangé pour procéder à un calibrage de la couleur, à une analyse de la couleur, à une analyse de la texture et à une détection de caractéristique.

3. Système selon la revendication 1, dans lequel ledit module d'analyse est arrangé pour procéder à une analyse des vaisseaux sanguins dans les trames d'images satisfaisantes.

4. Système selon la revendication 1, dans lequel ledit module d'analyse comprend un module d'analyse de motif de trou arrangé pour procéder à une analyse des motifs de trous dans les trames d'images satisfaisantes.

5. Système selon la revendication 1, comprenant en outre un module de modélisation tridimensionnelle et de reconstruction arrangé pour :
reconstruire une image fixe en trois dimensions dudit gros intestin à partir desdites trames d'images satisfaisantes pour former un modèle reconstruit, dans lequel ladite reconstruction est effectuée en temps réelle pendant ladite colonoscopie, procéder à une correction de la distorsion en barillet, à un calibrage de la géométrie, à une modélisation basée sur l'image et à un assemblage des données tridimensionnelles ; et
dans lequel ledit module d'analyse est en outre arrangé pour retrouver une forme tridimensionnelle et des informations de taille de toutes lésions ou tous polypes du gros intestin à partir dudit modèle reconstruit.
